(19) Europäisches Patentamt
European Patent Office
Office européen des brevets

(11) Veröffentlichungsnummer: **0 000 573**
**B1**

# (12) EUROPÄISCHE PATENTSCHRIFT

(45) Veröffentlichungstag der Patentschrift: **18.03.81**

(51) Int. Cl.³: **C 07 C 149/42** C08G18/10

(21) Anmeldenummer: **78100485.8**

(22) Anmeldetag: **24.07.78**

## (54) Verfahren zur Herstellung von Diaminen mit einer Thioäthergruppe.

(30) Priorität: **30.07.77 DE 2734575**

(43) Veröffentlichungstag der Anmeldung: **07.02.79 Patentblatt 79/3**

(45) Bekanntmachung des Hinweises auf die Patenterteilung: **18.03.81 Patentblatt 81/11**

(84) Benannte Vertragsstaaten: **BE DE FR GB**

(56) Entgegenhaltungen:
**GB-A-404 596**
**GB-A-1 161 915**
**US-A-1 954 706**
**US-A-2 791 612**
**US-A-3 789 072**
**US-A-3 950 542**
**JOURNAL OF PHARMACEUTICAL SCIENCES, Vol. 51, Nr. 9, September 1962, CHARLES DAVIS et al. »Synthesis of some N- and S-substituted derivates of 2-Aminobenzenethiol«, Seite 840—842**

(73) Patentinhaber: **BAYER AG, Zentralbereich Patente, Marken und Lizenzen, D-5090 Leverkusen 1, Bayerwerk (DE)**

(72) Erfinder: **Uhrhan, Paul, Dr., Brunnenweg 27, D-5068 Odenthal (DE)**
Erfinder: **Schwindt, Jürgen, Dr., Heymannstrasse 38, D-5090 Leverkusen 1 (DE)**
Erfinder: **Grögler, Gerhard, Dr., von Diergardt-Strasse 46, D-5090 Leverkusen 1 (DE)**

**EP 0 000 573 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

Verfahren zur Herstellung von Diaminen mit einer Thioäthergruppe

Die vorliegende Erfindung betrifft ein verbessertes Verfahren zur Herstellung von Diaminen der allgemeinen Formel (I)

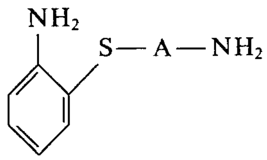

(I)

In dieser allgemeinen Formel (I) bedeutet A einen geradkettigen oder verzweigten, zweiwertigen, aliphatischen Kohlenwasserstoffrest mit 2—20, vorzugsweise 2—12, besonders bevorzugt 2—6 C-Atomen oder einen araliphatischen Kohlenwasserstoffrest mit 8 C-Atomen.

Aus der Literatur (Farmaco Ed. Sci. 22 (7), 519—527 [1967]) ist ein Verfahren zur Herstellung von Verbindungen der Formel (I), in welcher A für einen gegebenenfalls methylsubstituierten Äthylenrest steht, bekanntgeworden, wobei man o-Aminothiophenol im alkalischen Milieu mit den entsprechenden $\beta$-Halogenalkylaminen umsetzt. o-Aminothiophenol oder dessen Salze rufen jedoch auf der Haut empfindlicher Personen schwere Allergien hervor, was für ein technisches Herstellungsverfahren von großem Nachteil ist.

Gemäß US-Patent 3 789 072 werden Verbindungen analog zu jenen der obigen Formel (I), welche gegebenenfalls am aromatischen Ring substituiert sein können, die jedoch an der aliphatischen Aminogruppe alkyliert sind, durch Umsetzung eines o-Aminothiophenols mit einem N-alkylierten Halogenalkylamin in Gegenwart von $NaNH_2$ hergestellt.

Es wurde nun gefunden, daß man Verbindungen der allgemeinen Formel (I) auf einfache Weise und in hoher Ausbeute gefahrlos herstellen kann, indem man Benzothiazol in Gegenwart einer starken wäßrigen Base erhitzt und anschließend mit Halogenalkylaminen umsetzt. Es ist dabei als überraschend anzusehen, daß mit Hilfe des erfindungsgemäßen Verfahrens Produkte mit hohem Reinheitsgrad, d. h. ohne störende Nebenprodukte, erhalten werden, obwohl bekanntlich primäre und insbesondere primäre aliphatische Aminogruppen unter diesen Bedingungen leicht mit Verbindungen, die Alkylhalogenid-Strukturelemente aufweisen, unter N-Alkylierung reagieren.

Gegenstand der Erfindung ist ein Verfahren zur Herstellung von Diaminen der allgemeinen Formel

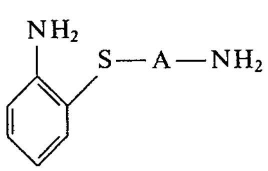

welches dadurch gekennzeichnet ist, daß man 1 Mol Benzothiazol bei 40—180°C mit einer mindestens äquivalenten Menge einer starken wäßrigen Base und anschließend, gegebenenfalls portionsweise, mit 0,1 bis 1 Mol, vorzugsweise 0,7 bis 1 Mol, besonders bevorzugt 1 Mol, eines Halogenalkylamins der allgemeinen Formel

$$X{-}A{-}NH_2$$

bzw. eines Salzes, eines Amids oder Urethans des Halogenalkylamins umsetzt und gegebenenfalls anschließend die Acyl- bzw. Urethangruppe von der aliphatischen Aminogruppe hydrolytisch abspaltet, wobei

A einen gegebenenfalls verzweigten, zweiwertigen, aliphatischen Kohlenwasserstoffrest mit 2—20, vorzugsweise 2—12, besonders bevorzugt 2—6 C-Atomen oder einen araliphatischen Kohlenwasserstoffrest mit 8 C-Atomen darstellt und

X für ein Halogenatom, vorzugsweise Chlor oder Brom, besonders bevorzugt Chlor, steht.

Beispielsweise steht A für einen Äthylen-, 1,2- oder 1,3-Propylen-, 1,2-Butylen-, 1,2-Isobutylen-, Tetramethylen-, 2,3-Butylen-, Pentamethylen-, 1,2-Pentylen-, 1,2-Isopentylen-, Hexamethylen-, 1,2-Hexylen-, Isobutyläthylen-, Octamethylen-, Dodekamethylen-, Xylylen- oder Phenyläthylen-Rest.

Verbindungen der obigen allgemeinen Formel (I), welche nach dem erfindungsgemäßen Verfahren erhältlich und zum Teil neu sind, sind z. B. die folgenden:

2-(2-Aminoäthylthio)-anilin,
2-(2-Aminopropylthio)-anilin,
2-(3-Aminopropylthio)-anilin,
2-(2-Amino-2,2-dimethyl-äthylthio)-anilin,
2-(4-Aminobutylthio)-anilin,
2-(2-Amino-1,2-dimethyl-äthylthio)-anilin,
2-(5-Aminopentylthio)-anilin,
2-(6-Aminohexylthio)-anilin,
2-(5-Aminohexylthio)-anilin,
2-(2-Aminoisobutylthio)-anilin,
2-(12-Aminododecylthio)-anilin und
2-(2-Aminophenyläthylthio)-anilin.

Im erfindungsgemäßen Verfahren werden Benzothiazol, sowie Halogenalkylamine der allgemeinen Formel

$$X{-}A{-}NH_2$$

bzw. Derivate davon als Ausgangsmaterialien eingesetzt.

Halogenalkylamine sind allgemein bekannte

Verbindungen, welche sich nach dem Fachmann geläufigen Verfahren leicht darstellen lassen. Erfindungsgemäß wird das Halogenalkylamin entweder als solches oder vorzugsweise in Form des Ammoniumsalzes mit einer organischen oder vorzugsweise anorganischen Säure oder in acylierter Form bzw. als Halogenalkylurethan eingesetzt. Der Acyl- bzw. Urethan-Rest wird im Verlauf bzw. am Ende der erfindungsgemäßen Umsetzung hydrolytisch abgespalten.

Bevorzugte Ammoniumsalze der Halogenalkylamine sind deren Hydrochloride. Geeignete Acyl-Reste sind geradkettige oder verzweigte aliphatische Acyl-Reste mit 1—18 C-Atomen oder aromatische Acyl-Reste mit 6—10 C-Atomen, wobei der Acetyl-Rest bevorzugt ist. Geeignete Urethane entsprechen der allgemeinen Formel

$$X—A—NH—CO_2R$$

in der

R einen geradkettigen oder verzweigten aliphatischen Rest mit 1—12 C-Atomen oder einen gegebenenfalls durch Halogen-Atome und/oder Alkyl-Gruppen ($C_1—C_4$) substituierten aromatischen Rest mit 6—10 C-Atomen bedeutet. Vorzugsweise steht R für den tert.-Butyl-Rest.

Im Anschluß an die erfindungsgemäße Reaktion können die Acyl- bzw. Urethanreste auf einfache Weise nach bekannten Verfahren hydrolytisch abgespalten werden.

Erfindungsgemäß versetzt man zunächst Benzothiazol mit der stöchiometrischen Menge oder einem Überschuß (vorzugsweise 0 bis 10% Überschuß) einer starken wäßrigen Base, bevorzugt einer Alkali- oder Erdalkalilauge (vorzugsweise Natron- oder Kalilauge, besonders bevorzugt Natronlauge), erhitzt die Mischung, bis eine klare Lösung entstanden ist (dies dauert im allgemeinen etwa 1/2 bis 4 Stunden) und fügt das Halogenalkylamin bzw. dessen Derivat anschließend, vorzugsweise in Form einer Lösung in einem geeigneten Lösungsmittel, zu dem Reaktionsansatz hinzu. Die Reaktionspartner werden vorzugsweise in stöchiometrischen Mengen miteinander umgesetzt. Man kann jedoch auch mit einem bis zu 10fachen Überschuß an Benzothiazol arbeiten. Diese zweite Stufe der erfindungsgemäßen Reaktion dauert (je nach Reaktionstemperatur und Reaktivität des Halogenalkylamins) ca. 1/2 bis 10 h.

Als Lösungsmittel können bei dem erfindungsgemäßen Verfahren beispielsweise die folgenden eingesetzt werden:

Wasser, Alkohole wie z. B. Methylalkohol, Äthylalkohol, Propylalkohol, Isopropylalkohol und Butylalkohol, Ketone wie Aceton oder Methyläthylketon, Äthylenglykol und dessen Alkyläther, Diäthylenglykol und Triäthylenglykol, Dimethylformamid, Dimethylsulfoxid, Dioxan usw. Auch Mischungen solcher Lösungsmittel sind geeignet. Bevorzugte Lösungsmittel sind Wasser und niedere Alkohole; ganz besonders bevorzugt sind Wasser sowie Mischungen von Wasser mit Methyl-, Äthyl- oder Isopropylalkohol.

Pro Mol Benzothiazol verwendet man vorzugsweise 200 bis 5000 ml Lösungsmittel, besonders bevorzugt 200 bis 2000 ml Lösungsmittel.

Die Reaktionstemperatur liegt im Bereich von 40 bis 180°C, vorzugsweise zwischen 50 und 140°C, wobei der Bereich von 70 bis 120°C besonders bevorzugt ist.

Die Gesamtreaktionszeit beträgt im allgemeinen ca. 1 Stunde bis 10 Stunden, vorzugsweise 3 bis 6 Stunden.

Der Reaktionsdruck beträgt in der Regel 1 bar bis 10 bar. Vorzugsweise wird bei Normaldruck gearbeitet; jedoch kann es bei träge reagierenden Halogenalkylaminen auch vorteilhaft sein, zur Erreichung einer größeren Reaktionsgeschwindigkeit bei erhöhtem Druck zu arbeiten.

Die 2-(ω-Aminoalkylthio)-aniline scheiden sich nach dem Alkalischstellen des Reaktionsansatzes als Flüssigkeiten ab und können gegebenenfalls im Vakuum destilliert werden, wenngleich dies im allgemeinen nicht erforderlich ist. Auch die Reinigung durch Überführung der 2-(ω-Aminoalkylthio)-aniline in deren Hydrochloride mit HCl ist möglich aber nicht erforderlich, da man die freien Amine nach dem erfindungsgemäßen Verfahren in hohen Ausbeuten und in überraschend hoher Reinheit (meist über 98%) erhält.

Die erfindungsgemäß zugänglichen Verbindungen der allgemeinen Formel (I) sind wertvolle Zwischenprodukte bei der an sich bekannten Herstellung von Polyurethankunststoffen aus Polyisocyanaten, höhermolekularen Polyhydroxylverbindungen und Diaminen als Kettenverlängerungsmitteln.

Als Kettenverlängerungsmittel besonders gut geeignet sind in vielen Fällen jene erfindungsgemäß zugänglichen Diamine, bei welchen die aliphatische Aminogruppen durch mindestens 3 C-Atome vom Schwefelatom getrennt ist. Diese Diamine zeichnen sich gegenüber jenen mit nur 2 C-Atomen nur 2 C-Atomen zwischen Schwefelatom und Aminogruppe durch eine stark unterschiedliche Reaktivität von aromatischer und aliphatischer Aminogruppe aus (siehe Beispiel 6), was insbesondere dann von Vorteil ist, wenn beim Aufbau eines Polyurethanharnstoffs ein Zwischenprodukt mit endständigen Aminogruppen hergestellt werden soll, wobei das Polyisocyanat gezielt nur mit einer der beiden Aminogruppen des Kettenverlängerungsmittels reagieren soll. In diesen Diaminen, der allgemeinen Formel

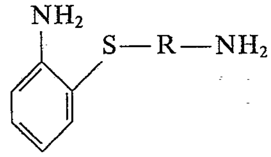

steht R für einen gegebenenfalls verzweigten zweiwertigen aliphatischen Rest mit 3 bis 20, vorzugsweise 3 bis 12, besonders bevorzugt 3 bis

6 C-Atomen, mit der Maßgabe, daß die aliphatische Aminogruppe vom Schwefelatom durch mindestens 3 C-Atome getrennt ist, oder einen zweiwertigen araliphatischen Rest mit 8 C-Atomen. Besonders bevorzugt stellt R einen linearen Alkylrest mit 3 bis 6 C-Atomen dar.

Die nachfolgenden Beispiele dienen zur näheren Erläuterung der Erfindung. Alle Mengenangaben beziehen sich, wenn nicht anders vermerkt, auf Gewichtsteile. Die Struktur der Substanzen wurde durch ihre IR-, NMR- und Massenspektren sowie durch Elementaranalyse eindeutig gesichert.

Beispiel 1
2-(2-Aminoäthylthio)-anilin

135 Teile Benzothiazol und 80 Teile Natriumhydroxid werden in 200 Teilen Wasser 3 Stunden unter Rückfluß gekocht. Nach dem Abkühlen auf 70° C werden 200 Teile Methanol hinzugefügt und bei 70°C innerhalb von 2 Stunden eine Lösung von 116 Teilen Chloräthylamin-hydrochlorid in 200 Teilen Wasser in den Ansatz getropft. Man rührt weitere 3 Stunden unter Rückfluß, kühlt ab und versetzt die Mischung mit einer Lösung von 50 Teilen Natriumhydroxid in 100 Teilen Wasser. Dann wird die organische Phase abgetrennt und die wäßrige Phase zweimal mit je 100 Teilen Toluol extrahiert.

Die vereinigten organischen Phasen werden eingedampft. Man erhält 156 Teile 2-(2-Aminoäthylthio)-anilin in Form einer gelblichen Flüssigkeit vom Siedepunkt 152° C (0,08 mbar).

Elementaranalyse:
$C_8H_{12}N_2S$ (168,26)
Ber.: C 57,11 H 7,19 N 16,65 S 19,05
Gef.: C 57,05 H 7,30 N 16,48 S 19,00

Beispiel 2
2-(3-Aminopropylthio)-anilin

270 Teile Benzothiazol und 180 Teile Natriumhydroxid werden in 400 Teilen Wasser 3 Stunden unter Rückfluß gekocht. Nach dem Abkühlen auf 70° C werden 250 Teile Methanol hinzugefügt und bei 70°C innerhalb von 2 Stunden eine Lösung von 260 Teilen 3-Chlorpropylamin-hydrochlorid in 500 Teilen Wasser in den Ansatz getropft. Nachdem man weitere 3 Stunden bei 100°C nachgerührt hat, wird abgekühlt und eine Lösung von 100 Teilen Natriumhydroxid in 200 Teilen Wasser hinzugefügt. Man trennt die organische Phase ab, wäscht die wäßrige Phase noch einmal mit je 200 Teilen Toluol und dampft die vereinigten organischen Phasen ein.

Es verbleiben 346 Teile 2-(3-Aminopropylthio)-anilin als gelbliche Flüssigkeit vom Siedepunkt 130° C (0,053 mbar).

Elementaranalyse:
$C_9H_{14}H_2S$ (182,29)
Ber.: C 59,30 H 7,74 N 15,37 S 17,59
Gef.: C 59,15 H 7,83 N 15,51 S 17,40

Beispiel 3
2-(2-Aminopropylthio)-anilin

135 Teile Benzothiazol und 80 Teile Natriumhydroxid werden in 200 Teilen Wasser 3 Stunden unter Rückfluß gekocht. Nach dem Abkühlen auf 80° C fügt man 200 Teile Äthanol hinzu und tropft bei 80° C eine Lösung von 130 Teilen 2-Chlorpropylamin-hydrochlorid in 300 Teilen Wasser innerhalb von 2 Stunden in den Ansatz. Man rührt weitere 3 Stunden unter Rückfluß, kühlt ab und versetzt die Mischung mit einer Lösung von 50 Teilen Natriumhydroxid in 100 Teilen Wasser. Dann wird die organische Phase abgetrennt, die wäßrige Phase zweimal mit je 100 Teilen Toluol extrahiert und die vereinigten organischen Phasen eingedampft. Es verbleiben 170 Teile 2-(2-Aminopropylthio)-anilin als gelbliche Flüssigkeit vom Siedepunkt 137° C (0,08 mbar).

Elementaranalyse:
$C_9H_{14}N_2S$
Ber.: C 59,30 H 7,74 N 15,37 S 17,59
Gef.: C 59,41 H 7,65 N 15,31 S 17,70

Beispiel 4
2-(6-Aminohexylthio)-anilin

135 Teile Benzothiazol und 80 Teile Natriumhydroxid werden in 200 Teilen Wasser 3 Stunden unter Rückfluß gekocht. Nach dem Abkühlen auf 60°C werden 600 Teile Methanol eingetragen und bei 70° C 224,7 Teile N-(6-Chlorhexyl)-O-tert.-butylurethan innerhalb von 2 Stunden zugetropft. Nachdem man weitere 3 Stunden bei 70° C nachgerührt hat, kühlt man ab und trennt die organische Phase ab. Die wäßrige Phase wird noch zweimal mit je 150 Teilen Toluol extrahiert und die vereinigten organischen Phasen anschließend eingedampft. Es verbleiben 308 Teile einer gelben, viskosen Flüssigkeit, die in 950 Teile konzentrierter Salzsäure eingerührt werden. Man kocht zwei Stunden unter Rückfluß, kühlt ab und macht mit Natronlauge alkalisch. Anschließend wird die organische Phase abgetrennt, die wäßrige Phase noch zweimal mit je 200 Teilen Toluol extrahiert und die vereinigten organischen Phasen eingedampft. Es verbleiben 210 Teile 2-(6-Aminohexylthio)-anilin als gelbliche Flüssigkeit vom Siedepunkt 145° C (0,053 mbar).

Elementaranalyse:
$C_{12}H_{20}N_2S$ (224,37)
Ber.: C 64,24 H 8,99 N 12,49 S 14,29
Gef.: C 64,10 H 9,13 N 12,40 S 14,31

Beispiel 5
Verwendung der nach dem erfindungsgemäßen Verfahren erhaltenen Diamine zur Herstellung elastischer Polyurethankunststoffe

100 Teile eines Präpolymeren (NCO-Gehalt: 3,6 Gew.-%), hergestellt aus einem linearen Polypropylenglykol (Molekulargewicht: 2000; OH-Zahl: 56) und 2,4-Toluylen-diisocyanat werden bei einem Äquivalentverhältnis NCO : $NH_2$ von 1,1 : 1 mit 6,5 bis 8,65 Teilen der nach Beispiel 1 bis 4 gewonnenen araliphatischen schwefelhaltigen Diamine innerhalb von 30 Sekunden gut vermischt, das reagierende Gemisch in eine vorgeheizte Form gegeben und der Elastomerkörper bei 110°C 24 Stunden lang ausgeheizt. Man erhält Elastomerkörper von ausgezeichnetem mechanischen Werteniveau.

Beispiel 6

Untersuchung der Reaktivität von Diaminen gemäß Beispiel 1, 2 und 4 gegenüber Isocyanaten

a) in eine Lösung von 0,1 Mol 2(2-Aminoäthylthio)-anilin in 50 g Chlorbenzol werden bei 10°C 17,0 g (0,2 Mol) Propylisocyanat gegeben. Der NCO-Gehalt der Lösung beträgt 9,3%. Das Reaktionsgemisch erwärmt sich auf 63°C. Eine nach 30 Sekunden durchgeführte NCO-Bestimmung ergibt 4,12% NCO. Nach weiteren 10 Minuten können noch 1,8% NCO nachgewiesen werden.
b) Analog wie unter a) beschrieben, werden 0,1 Mol 2-(3-Aminopropylthio)-anilin und 0,2 Mol Propylisocyanat umgesetzt. Der Anfangsgehalt an NCO-Gruppen beträgt 9,1%; das Reaktionsgemisch erwärmt sich auf 60°C. Nach 30 Sekunden sind noch 4,0% NCO, nach 15 Minuten noch 3,95% NCO und nach 45 Minuten 3,6% NCO durch Titration nachzuweisen.
c) Analog wie unter a) beschrieben, werden 0,1 Mol 2-(6-Aminohexylthio)-anilin und 0,2 Mol Propylisocyanat umgesetzt. Der Anfangs-NCO-Gehalt beträgt 8,65%. Das Reaktionsgemisch erwärmt sich nach Zugabe des Isocyanats auf 62°C. Nach 30 Sekunden sind noch 4,1% NCO, nach 15 Minuten 3,8% und nach 105 Minuten noch 3,4% an NCO-Gruppen nachzuweisen.

Die Versuche zeigen, daß bei den erfindungsgemäßen Diaminen aus Beispiel 2 und 4 die aromatische Aminogruppe gegenüber Isocyanaten wesentlich weniger reaktiv ist als die aliphatische.

**Patentansprüche**

1. Verfahren zur Herstellung von Diaminen der allgemeinen Formel

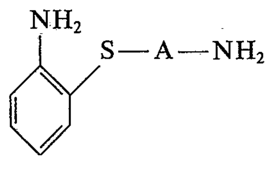

dadurch gekennzeichnet, daß man 1 Mol Benzothiazol bei 40 bis 180°C mit einer mindestens äquivalenten Menge einer starken wäßrigen Base und anschließend, gegebenenfalls portionsweise, mit 0,1 bis 1 Mol eines Halogenalkylamins der allgemeinen Formel

$$X-A-NH_2$$

bzw. eines Salzes, eines Amids oder Urethans des Halogenalkylamins umsetzt, und gegebenenfalls die Acyl- bzw. Urethangruppe von der aliphatischen Aminogruppe hydrolytisch abspaltet, wobei

A einen gegebenenfalls verzweigten, zweiwertigen aliphatischen Kohlenwasserstoffrest mit 2—20 C-Atomen oder einen araliphatischen Kohlenwasserstoffrest mit 8 C-Atomen darstellt und
X für ein Halogenatom steht.

2. Verfahren nach Anspruch 1, dadurch gekennzeichnet, daß

A einen aliphatischen Kohlenwasserstoffrest mit 2—12 C-Atomen darstellt und
X für Chlor oder Brom steht.

3. Verfahren nach Anspruch 1, dadurch gekennzeichnet, daß

A einen aliphatischen Kohlenwasserstoffrest mit 2—6 C-Atomen darstellt und
X für Chlor steht.

4. Verfahren nach Anspruch 1 bis 3, dadurch gekennzeichnet, daß in Gegenwart von 0,2 bis 5 l eines Lösungsmittels, bezogen auf 1 Mol Benzothiazol, gearbeitet wird.

5. Verfahren nach Anspruch 1 bis 4, dadurch gekennzeichnet, daß Wasser, Methanol, Äthanol, Isopropanol oder Gemische daraus als Lösungsmittel verwendet werden.

6. Verfahren nach Anspruch 1 bis 5, dadurch gekennzeichnet, daß das Hydrochlorid oder das tert.-Butylurethan des Halogenalkylamins eingesetzt werden.

7. Verfahren nach Anspruch 1 bis 6, dadurch gekennzeichnet, daß 0,7 bis 1 Mol des Halogenalkylamins bzw. seines Derivats pro 1 Mol Benzothiazol eingesetzt werden.

**Claims**

1. A process for the production of diamines of the general formula

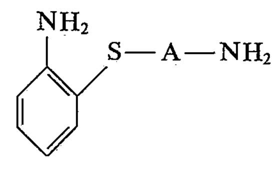

characterised in that 1 mole of benzothiazole is

reacted, at from 40 to 180°C, with an at least equivalent quantity of a strong aqueous base and then, optionally in portions, with from 0.1 to 1 mole of a halogen alkylamine of the general formula:

$$X-A-NH_2$$

or of a salt, an amide or a urethane of the halogen alkylamine, and the acyl or urethane group is optionally split off hydrolytically from the aliphatic amino group; in the above formulae:

A represents an optionally branched, difunctional aliphatic hydrocarbon radical containing from 2 to 20 carbon atoms or an araliphatic hydrocarbon radical containing 8 carbon atoms; and
X represents a halogen atom.

2. A process according to claim 1, characterised in that

A represents an aliphatic hydrocarbon radical containing from 2 to 12 carbon atoms; and
X represents chlorine or bromine.

3. A process according to claim 1, characterised in that

A represents an aliphatic hydrocarbon radical containing from 2 to 6 carbon atoms; and
X represents chlorine.

4. A process according to claim 1 to 3, characterised in that the reaction is carried out in the presence of from 0.2 to 5 litres of a solvent, based on 1 mole of benzothiazole.

5. A process according to claim 1 to 4, characterised in that water, methanol, ethanol, isopropanol or mixtures thereof are used as solvent.

6. A process according to claim 1 to 5, characterised in that the hydrochloride or the tert.-butyl urethane of the halogen alkylamine is used.

7. A process according to claim 1 to 6, characterised in that from 0.7 to 1 mole of the halogen alkylamine or its derivative is used per mole of benzothiazole.

**Revendications**

1. Procédé de fabrication de diamines de formule générale:

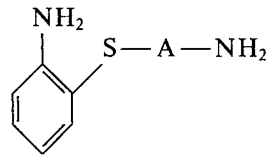

caractérisé en ce qu'on fait réagir 1 mole de benzothiazol à 40—180°C avec une quantité au moins équivalente d'une base aqueuse forte et ensuite, éventuellement par portions, avec 0,1 à 1 mole d'une halogénoalcoylamine de formule générale

$$X-A-NH_2$$

ou d'un sel, d'une amide ou d'un uréthane de l'halogénoalcoylamine et en ce qu'éventuellement on clive hydrolytiquement le groupe acyle ou uréthane du groupe aminé aliphatique,

A représentant un radical hydrocarboné aliphatique bivalent éventuellement ramifié ayant 2 à 20 atomes de carbone ou un radical hydrocarboné araliphatique ayant 8 atomes de carbone et
X représentant un atome d'halogène.

2. Procédé selon la revendication 1, caractérisé en ce que

A représente un radical hydrocarboné aliphatique ayant 2 à 12 atomes de carbone et
X du chlore ou du brome.

3. Procédé selon la revendication 1, caractérisé en ce que

A représente un radical hydrocarboné aliphatique ayant 2 à 6 atomes de carbone et
X du chlore.

4. Procédé selon l'une quelconque des revendications 1 à 3, caractérisé en ce qu'on opère en présence de 0,2 à 5 litres d'un solvant pour 1 mole de benzothiazol.

5. Procédé selon l'une quelconque des revendications 1 à 4, caractérisé en ce qu'on utilise de l'eau, du méthanol, de l'éthanol, de l'isopropanol ou des mélanges de ceux-ci comme solvant.

6. Procédé selon l'une quelconque des revendications 1 à 5, caractérisé en ce qu'on utilise le chlorhydrate ou l'uréthane de t-butyle de l'halogénoalcoylamine.

7. Procédé selon l'une quelconque des revendications 1 à 6, caractérisé en ce qu'on utilise 0,7 à 1 mole de l'halogénoalcoylamine ou de son dérivé pour 1 mole de benzothiazol.